Europäisches Patentamt

European Patent Office

Office européen des brevets

(11) Publication number: **0 439 858 A1**

# EUROPEAN PATENT APPLICATION

(21) Application number: **90203431.3**

(22) Date of filing: **18.12.90**

(51) Int. Cl.⁵: **A61K 31/415, A61K 9/20**

(30) Priority: **31.01.90 NL 9000238**

(43) Date of publication of application:
**07.08.91 Bulletin 91/32**

(84) Designated Contracting States:
**AT BE CH DE DK ES FR GB GR IT LI LU NL SE**

(71) Applicant: **AESCULAAP B.V.**
**Mijlstraat 35**
**NL-5281 LJ Boxtel(NL)**

(72) Inventor: **Tukker, Jozef Jacobus**
**Croesestraat 79**
**NL-3522 AD Utrecht(NL)**

(74) Representative: **Boelsma, Gerben Harm, Ir. et al**
**Octrooibureau Polak & Charlouis Laan**
**Copes van Cattenburch 80**
**NL-2585 GD Den Haag(NL)**

(54) **Veterinary phenytoin composition.**

(57) A veterinary sustained release phenytoin composition is provided which, calculated as free phenytoin, contains at least 450 mg of active substance

## VETERINARY PHENYTOIN COMPOSITION

The invention relates to a veterinary phenytoin composition.

Phenytoin or 4,4-diphenylhydantoin is anti-epileptic which is already in use for decades. In principle the phenytoin may be used as free compound or as a salt or other derivative, for instance the sodium salt, calcium salt or 3-valerate. Commercial preparations contain either the sodium salt or the free compound. In the human therapy the preparation has been well established. According to the Information Medicamentorum half-life values between 9 and 122 hours have been reported in humans. This means that for human therapy there is in principle no need of compositions having a prolonged activity.

In the veterinary medicine epilepsy is in particular a problem with dogs. Up till now, the normal human preparations were used for dogs, because the various forms of epilepsy in dogs corresponds to those in humans. However, there appears to exist an important difference between the metabolism in the case of dogs and of humans. Thus, in dogs plasma half-life values of 2 to no more than 7 hours found for the phenytoin, the 7 hours being an exception. One should add thereto that the duration of the activity of every administered dose decreases with time, because the dogs will decompose the agent quicker every time due to enzymatic reaction, a phenomenon, which is also called auto-induction. Just like in the case of humans the medicine has to be used during the whole life of epileptic dogs and the ever decreasing duration of the activity means accordingly that finally one has to administer to the dog a tablet or capsule a number of times per day, sometimes even 5 times a day. Both for the attendant and the dog this is a disagreeable matter.

The U.S.P.(XXI, pages 831-32) describes extended phenytoin sodium capsules, but not less than 70 % thereof is dissolved in water within 120 minutes. According to Chem.Abstr., Vol. 108 (1988), page 314, ref. nr. 82000k practically the same holds true for phenytoin sodium sustained release tablets. As appears from the stated dissolution rates, such preparations are not sustained release preparations in the true sense of the word, and, as will be clear, the preparations for human therapy would not offer any solution for the present problem.

It has now been found that the above discussed problem with dogs can be solved with the aid of a preparation having delayed release. Such a preparation can be prepared according to the usual techniques.

Consequently, the invention provides a veterinary phenytoin preparation which is characterized in that it is a preparation having sustained release which, calculated as free phenytoin, contains at least 450 mg of active substance.

The preparations of the invention can be solid or liquid, for instance thickened suspensions, capsules, coated tablets or tablets. For the sake of simplicity of administration tablets are used preferably. Therefore the invention is described hereinbelow in the first place with respect to the manufacture and use of tablets, it being understood that the invention is not restricted thereto.

Whereas in human therapy it is possible to use microcrystalline phenytoin as an alternative for the sodium salt, which leads to a finely divided precipitate of the phenytoin itself in the stomic (vide again the Informatorium Medicamentorum), it is possible to use the phenytoin in the free form without further measures in the preparation of the invention, due to the gradual setting free thereof from the tablet. Accordingly, one now has the choice between use of the compound in the free form and in the form of the sodium salt, calcium salt, 3-valerate or other salts, the first mentioned form being preferred.

Sustained release preparations, particularly tablets, can be prepared in a manner known per se, for instance with the aid of gel-forming agents, and wherein of course the composition can contain also further adjuvants, if desired.

As gel-forming agents in the first place cellulose and starch derivatives enter into consideration, and furthermore also substances like polyvinylpyrrolidone, polyvinylalcohol, gelatine, tragacanth gum, gum arabic and inorganic gel-forming thickening agents. Particularly suitable gel-forming agents are hydroxypropyl-methyl cellulose, hydroxypropyl cellulose and carboxy-methyl cellulose. With the aid thereof the tablets or other preparations of the invention can be manufactured in a manner known to the expert.

Apart from the active substance and the gel-former the tablets of the invention may contain further adjuvants, as desired, e.g. fillers, lubricants, such as magnesium stearate, colouring agents, binders and aroma's and flavorants.

In order to mask the taste of the phenytoin which is disagreable to the animals, the tablets furthermore can be provided a coating layer wherein also a flavorant can be incorporated again.

As mentioned already, the preparation of the invention has an active substance content of at least 450 mg, calculated as free phenytoin. It is not well possible to indicate an upper limit for the content of active substance, but for practical purposes it will not exceed 2000 mg per unit dosage. Preferably, the preparation of the invention contains a content of active substance of 450-900 mg, calculated as free phenytoin. With this preferred dosage of 450-900 mg a sufficient quantity of the medicine (expressed in mg/kg) can be administerd to dogs having a weight of e.g. 10-20 kg. For smaller animals the tablets, if desired, can be made dividable, for instance with the aid of a groove.

Example

In order to prepare 2500 sustained release tablets having an active substance content of 645 mg pro tablet, 1612.5 g of phenytoin is mixed with 522.5 g of hydroxypropyl-methyl cellulose of 4000 mPa.s. Subsequently, a granulating liquid is prepared by dissolving 2.5 g of this same hydroxypropyl-methyl cellulose in 247.5 ml of water. One adds this to the first mentioned mixture and granulates with addition of 600 ml of water. Thereafter, the granulate is dried at a maximum temperature of 60 °C during 60 minutes, whereafter the material still contains 3.5-4 % of moisture. The granulate is milled and passed through a sieve having a mesh size of 1 mm, followed by the addition of 37.5 g of magnesium stearate and mixing again. The entire mixture of 2175 g is then processed to biconcave tablets having a weight of 870 mg.

When these tablets were administered to 6 dogs, a good constant therapeutic blood level was reached after about 3 hours and this blood level was maintained during several hours. After regular administration during several weeks the time of the constant blood level had not changed significantly. The average results of this experiment have been rendered in the added drawing.

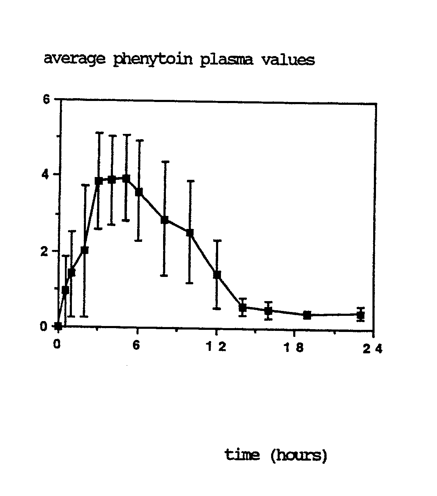

**Claims**

1. Veterinary phenytoin composition, characterized in that it is a sustained release preparation which, calculated as free phenytoin, contains at least 450 mg of active substance.

2. Composition according to claim 1, characterized in that it contains as the active substance the phenytoin as free compound.

3. Composition according to claim 1, characterized in that it contains the sodiun salt of phenytoin as the active substance.

4. Composition according to any of claims 1-3, characterized in that it is a tablet.

5. Composition according to claim 4, characterized in that the tablet is provided with a coating.

6. Composition according to any of claims 1-5, characterized in that it contains a gel former and, if desired, further adjuvants.

7. Composition according to any of claims 1-6, characterized that it contains as the gel former a cellulose or starch derivative, polyvinylpyrrolidone or polyvinylalcohol.

8. Composition according to claim 7, characterized in that it contains as the gel former hydroxypropyl-methyl cellulose, hydroxypropyl cellulose or carboxy-methyl cellulose.

**Claims for the following Contracting States: GR and SP.**

1. A process for preparing a veterinary phenytoin composition, characterized in that a sustained release preparation is prepared which, calculated as free phenytoin, contains at least 450 mg of active substance.

2. A process according to claim 1, characterized by using phenytoin in the free form as active substance.

3. A process according to claim 1, characterized by using the sodium salt of phenytoin as the active substance.

4. A process according to any of claims 1-3, characterized in that a tablet is prepared.

5. A process according to claim 4, characterized in that the tablet is provided with a coating.

6. A process according to any of claims 1-5, characterized by using therein a gel-forming agent, and, if desired, further adjuvants.

7. A process aacording to any of claims 1-6, characterized by using as the gel-forming agent a cellulose or starch derivative, polyvinylpyrrolidone or polyvinyl alcohol.

8. A process according to claim 7, characterized by using as the gel-forming agent hydroxypropyl-methyl cellulose, hydroxypropyl cellulose or carboxymethyl cellulose

average phenytoin plasma values

time (hours)

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | BIOLOGICAL ABSTRACTS, vol.67, no. 11, 1979, abstract no. 69053, Philadelphia, PA, US; SEKIKAWA H. et al.:"Dissolution behaviors and gastro intestinal absorption of phenytoin in phenytoin poly vinyl pyrrolidone co precipitate" & CHEM. PHARM. BULL. (TOKYO) 26(10): 3033-3039. 1978 * abstract * | 1-8 | A 61 K 31/415 A 61 K 9/20 |
| A | CHEMICAL ABSTRACTS, vol. 108, no. 10, 1988 Columbus, Ohio, USA ZHANG, YING et al.: "DISSOLUTION RATE STUDY ONPHENYTOIN SODIUM SUSTAINED RELEASE TABLETS" page 437; ref. no. 82000K * abstract * | 1-8 | |
| A | CHEMICAL ABSTRACTS, vol. 103, no. 12, 1985 Columbus, Ohio, USA HIERFOLZER, P. et al.: "USE OF ETHYL CELLULOSE FOR PRODUCTION OF SUSTAINED-RELEASE TABLETS WITH INSOLUBLE MATRIX; APPLICATION TO A SLIGHTLY SOLUBLE ACTIVE PRINCIPLE" page 314; ref. no. 92741P * abstract * | 1-8 | |

TECHNICAL FIELDS
SEARCHED (Int. Cl.5)

A 61 K

The present search report has been drawn up for all claims

| Place of search | Date of completion of search | Examiner |
|---|---|---|
| The Hague | 13 May 91 | LEHERTE C.F.M. |